# EUROPEAN PATENT APPLICATION

(11) **EP 4 269 588 A1**
(43) Date of publication of application: **01.11.2023**
(21) Application number: 21910848.7
(22) Date of filing: 22.12.2021
(51) Int. Cl.: C12N 15/13, A61K 39/395, A61P 25/28, C07K 16/28, C12N 1/15, C12N 1/19, C12N 1/21, C12N 5/10, C12N 15/63, C12P 21/08

(54) **ANTI-EPHA4 ANTIBODY**

(30) Priority: 24.12.2020 JP 2020214958
(71) Applicant: Eisai R&D Management Co., Ltd, Bunkyo-ku, Tokyo 112-8088 (JP)
(72) Inventor: KAWAKATSU, Tomomi, Kobe-shi, Hyogo 650-0047 (JP); YAMADA, Akio, Kobe-shi, Hyogo 650-0047 (JP); NAKATANI, Aki, Kobe-shi, Hyogo 650-0047 (JP); INOUE, Eiji, Kobe-shi, Hyogo 650-0047 (JP)
(74) Representative: Hoffmann Eitle
(86) International application number: PCT/JP2021/047534
(87) International publication number: WO 2022/138708

(57) **Abstract**

Provided is an anti-EphA4 antibody capable of binding to EphA4 and enhancing the cleavage of EphA4, and a pharmaceutical composition comprising the antibody as an active ingredient. The anti-EphA4 antibody comprises a heavy chain comprising a heavy chain CDR1 of SEQ ID NO: 30; a heavy chain CDR2 of SEQ ID NO: 31; and a heavy chain CDR3 of SEQ ID NO: 32; and a light chain comprising a light chain CDR1 of SEQ ID NO: 33; a light chain CDR2 of SEQ ID NO: 34; and a light chain CDR3 of SEQ ID NO: 35, or a heavy chain comprising a heavy chain CDR1 of SEQ ID NO: 42; a heavy chain CDR2 of SEQ ID NO: 31; and a heavy chain CDR3 of SEQ ID NO: 43; and a light chain comprising a light chain CDR1 of SEQ ID NO: 44; a light chain CDR2 of SEQ ID NO: 34; and a light chain CDR3 of SEQ ID NO: 35.

## Description

### [Technical Field]

The present disclosure relates to an antibody that binds to EphA4, a nucleic acid encoding the antibody, a vector comprising the nucleic acid, a cell comprising the vector, a method for producing the antibody, and a pharmaceutical composition comprising the antibody.

### [Background Art]

EphA4 is a member of the receptor tyrosine kinase family. Ephrin type A and type B are known as ligands of EphA4. Upon binding of EphA4 to its ligand ephrin, de-adhesion signals are induced. The involvement of EphA4 in the pathological condition of Alzheimer's disease (hereinafter, also referred to as "AD") has previously been suggested (Non Patent Literatures 1 to 4). It has been reported that the inhibition of the binding between EphA4 and ephrin rescues the amyloid β (Aβ)₍₁₋₄₂₎ oligomer-mediated functional impairment of neurotransmission (Patent Literature 1). In AD, aggregates (neurofibrillary tangles) formed by excessively phosphorylated tau are considered to be involved in neuronal cell death (Non Patent Literature 5). It has also been reported that the suppression of tau phosphorylation suppresses the neurodegeneration of synaptic loss (Non Patent Literatures 6 and 7) and ameliorates memory deficit or cognitive disfunction (Non Patent Literatures 8 to 11). There are reports suggesting the activation of CDK5 as a cause of tau phosphorylation (Non Patent Literatures 12 and 13).

EphA4 is highly expressed in the hippocampus and the cerebral cortex and cleaved by matrix metalloproteinase (MMP), ADAM (a disintegrin and metalloproteinase) and γ-secretase in a neural activity-dependent manner. This cleavage reaction of EphA4 is known to stabilize spines, which are important structures for neural functions (Non Patent Literature 14). A decreased density of spines in AD has been reported (Non Patent Literature 15). Furthermore, decrease in the number of cleaved fragments of EphA4 has also been confirmed in AD of NFT stages V and VI, suggesting that the cleavage reaction of EphA4 is involved in the pathological condition of AD (Non Patent Literature 16). A decreased spine density is known to correlate with cognitive disfunction, which is a clinical symptom of AD (Non Patent Literatures 15 and 17). Furthermore, it has also been reported that the stabilization of spines (increase in spine density) ameliorates cognitive functions in Alzheimer's disease models (Non Patent Literature 18), suggesting that the stabilization of spines has a therapeutic potential for AD.

KYL peptide, compound 1, and the like are known as existing EphA4 inhibitors (Patent Literature 2, Non Patent Literature 19 and Non Patent Literature 20). However, there has been no report on an antibody having activity of enhancing the cleavage of EphA4.

### [Citation List]

### [Patent Literature]

[Patent Literature 1] WO2016/019280A1
[Patent Literature 2] WO2012/156351A1

### [Non Patent Literature]

[Non Patent Literature 1] Vargas LM et al.,, PLoS One. 2014 Mar 21;9(3)
[Non Patent Literature 2] Fu AK et al., Proc Natl Acad Sci USA. 2014 Jul 8; 111(27): 9959-64
[Non Patent Literature 3] Rosenberger AF et al., Acta Neuropathol Commun. 2014 Jul 16; 2: 79
[Non Patent Literature 4] Huang TY et al., J Exp Med. 2017 Dec 4; 214(12): 3669-3685.
[Non Patent Literature 5] SantaCruz et al., Science. 2005 Jul 15; 309(5733): 476-81
[Non Patent Literature 6] Seo J et al., J Neurosci. 2017 Oct 11; 37(41): 9917-9924
[Non Patent Literature 7] Patrick GN et al., Nature. 1999 Dec 9; 402(6762): 615-22.
[Non Patent Literature 8] Onishi T et al., J Neurochem. 2011 Dec; 119(6): 1330-40
[Non Patent Literature 9] Belfiore R et al., Aging Cell. 2019 Feb; 18(1): e12873.
[Non Patent Literature 10] Webster SJ et al., Front Genet. 2014 Apr 23; 5: 88.
[Non Patent Literature 11] Grayson B et al., Behav Brain Res. 2015 May 15; 285: 176-93.
[Non Patent Literature 12] Cancino GI et al., Neurobiol Aging. 2011 Jul; 32(7): 1249-61.
[Non Patent Literature 13] Vargas LM et al., Biochim Biophys Acta Mol Basis Dis. 2018 Apr; 1864: 1148-1159.
[Non Patent Literature 14] Inoue E et al., J Cell Biol. 2009 May 4; 185(3): 551-64
[Non Patent Literature 15] Boros et al., Ann Neurol. 2017 Oct; 82(4): 602-614
[Non Patent Literature 16] Matsui C et al., Brain Pathol. 2012 Nov; 22(6): 776-87. doi: 10.1111/j.1750-3639
[Non Patent Literature 17] Akrama A et al., Neurobiol Aging. 2008 Sep; 29(9): 1296-1307
[Non Patent Literature 18] Suzuki K et al., Science. 2020 Aug 28; 369(6507): eabb4853
[Non Patent Literature 19] Goldshmit et al., PLoS one. 2011; 6(9): e24636
[Non Patent Literature 20] Van Hoecke et al., Nature Medicine. 2012 Sep; 18(9): 1418-22, 2012

### [Summary of Invention]

### [Technical Problem]

An object of the present disclosure is to provide an anti-EphA4 antibody capable of binding to EphA4 and enhancing the cleavage of EphA4, and a pharmaceutical composition comprising the antibody as an active ingredient.

### [Solution to Problem]

The present inventors have conducted diligent studies to attain the object and consequently completed the anti-EphA4 antibody of interest capable of binding to EphA4 and enhancing the cleavage of EphA4.

(1) An anti-EphA4 antibody comprising
   a heavy chain comprising
      (a) a heavy chain CDR1 consisting of the amino acid sequence shown in SEQ ID NO: 30;
      (b) a heavy chain CDR2 consisting of the amino acid sequence shown in SEQ ID NO: 31; and
      (c) a heavy chain CDR3 consisting of the amino acid sequence shown in SEQ ID NO: 32; and
   a light chain comprising
      (d) a light chain CDR1 consisting of the amino acid sequence shown in SEQ ID NO: 33;
      (e) a light chain CDR2 consisting of the amino acid sequence shown in SEQ ID NO: 34; and
      (f) a light chain CDR3 consisting of the amino acid sequence shown in SEQ ID NO: 35, or
   a heavy chain comprising
      (g) a heavy chain CDR1 consisting of the amino acid sequence shown in SEQ ID NO: 42;
      (h) a heavy chain CDR2 consisting of the amino acid sequence shown in SEQ ID NO: 31; and
      (i) a heavy chain CDR3 consisting of the amino acid sequence shown in SEQ ID NO: 43; and
   a light chain comprising
      (j) a light chain CDR1 consisting of the amino acid sequence shown in SEQ ID NO: 44;
      (k) a light chain CDR2 consisting of the amino acid sequence shown in SEQ ID NO: 34; and
      (l) a light chain CDR3 consisting of the amino acid sequence shown in SEQ ID NO: 35.
(2) The anti-EphA4 antibody according to (1), wherein
   the anti-EphA4 antibody is a human antibody.
(3) The anti-EphA4 antibody according to (1) or (2), wherein
   the anti-EphA4 antibody specifically binds to EphA4 and enhances the cleavage of EphA4.
(4) The anti-EphA4 antibody according to any of (1) to (3), wherein
   the anti-EphA4 antibody specifically binds to EphA4 and inhibits the binding between EphA4 and ephrin.
(5) The anti-EphA4 antibody according to any of (1) to (4), wherein
   the heavy chain comprises a variable region consisting of the amino acid sequence shown in SEQ ID NO: 7, and
   the light chain comprises a variable region consisting of the amino acid sequence shown in SEQ ID NO: 8.
(6) The anti-EphA4 antibody according to any of (1) to (5), wherein
   a constant region of the heavy chain and a constant region of the light chain each comprise an amino acid sequence derived from a human antibody.
(7) The anti-EphA4 antibody according to (6), wherein
   the constant region of the heavy chain is a constant region of human IgG.
(8) The anti-EphA4 antibody according to (7), wherein
   the constant region of the human IgG is a constant region of human IgG₂.
(9) The anti-EphA4 antibody according to (8), wherein
   the constant region of the human IgG₂ comprises the amino acid sequence shown in SEQ ID NO: 15.
(10) The anti-EphA4 antibody according to any of (1) to (4), wherein
   the heavy chain comprises a variable region consisting of the amino acid sequence shown in SEQ ID NO: 11, and
   the light chain comprises a variable region consisting of the amino acid sequence shown in SEQ ID NO: 12.
(11) The anti-EphA4 antibody according to any of (1) to (4) and (10), wherein
   a constant region of the heavy chain and a constant region of the light chain each comprise an amino acid sequence derived from a human antibody.
(12) The anti-EphA4 antibody according to (11), wherein
   the constant region of the heavy chain is a constant region of human IgG.
(13) The anti-EphA4 antibody according to (12), wherein
   the constant region of the human IgG is a constant region of human IgG consisting of a combination of human IgG₁ and human IgG₂.
(14) The anti-EphA4 antibody according to (13), wherein
   the constant region of the human IgG consisting of a combination of human IgG₁ and human IgG₂ comprises the amino acid sequence shown in SEQ ID NO: 16.
(15) The anti-EphA4 antibody according to any of (6) to (9) and (11) to (14), wherein
   the constant region of the light chain is a constant region of human Igλ.
(16) The anti-EphA4 antibody according to (15), wherein
   the constant region of the human Igλ comprises the amino acid sequence shown in SEQ ID NO: 17.
(17) An anti-EphA4 antibody comprising
   a heavy chain and a light chain, wherein
   the heavy chain comprises the amino acid sequence shown in SEQ ID NO: 20,
   the light chain comprises the amino acid sequence shown in SEQ ID NO: 21, and
   a C-terminal lysine of the heavy chain may be optionally deleted.
(18) The anti-EphA4 antibody according to (17), wherein
   the C-terminal lysine of the heavy chain is deleted.
(19) An anti-EphA4 antibody comprising
   a heavy chain and a light chain, wherein
   the heavy chain comprises the amino acid sequence shown in SEQ ID NO: 26,
   the light chain comprises the amino acid sequence shown in SEQ ID NO: 27, and
   a C-terminal lysine of the heavy chain may be optionally deleted.
(20) The anti-EphA4 antibody according to (19), wherein
   the C-terminal lysine of the heavy chain is deleted.
(21) An isolated nucleic acid encoding the anti-EphA4 antibody according to any of (1) to (20).
(22) A vector comprising the nucleic acid according to (21) .
(23) A host cell comprising the vector according to (22).
(24) A method for producing an anti-EphA4 antibody, comprising the step of culturing the host cell according to (23).
(25) A pharmaceutical composition comprising the anti-EphA4 antibody according to any of (1) to (20).
(26) The pharmaceutical composition according to (25), further comprising at least one pharmaceutically acceptable carrier.

### [Advantageous Effects of Invention]

The present disclosure provides an anti-EphA4 antibody capable of binding to EphA4 and enhancing the cleavage of EphA4, a nucleic acid encoding the antibody, a vector comprising the nucleic acid, a cell comprising the vector, a method for producing the antibody, and a pharmaceutical composition comprising the antibody as an active ingredient.

### [Brief Description of Drawings]

[Figure 1] Figure 1 shows the binding affinity of anti-EphA4 monoclonal antibodies (antibodies A and B) to human EphA4.
[Figure 2] Figure 2 shows the human EphA4-human ligand binding inhibitory activity of the anti-EphA4 monoclonal antibodies (antibodies A and B).
[Figure 3] Figure 3 shows the selectivity of the anti-EphA4 monoclonal antibodies (antibodies A and B) for each human Eph receptor.
[Figure 4] Figure 4 shows the reactivity of the anti-EphA4 monoclonal antibodies (antibodies A and B) with mouse, rat, monkey or human EphA4.
[Figure 5] Figure 5 shows the reactivity of the anti-EphA4 monoclonal antibodies (antibodies A and B) with a human EphA4 extracellular domain (ECD), ligand-binding domain (LBD), fibronectin type III domain 1 (FN1), and fibronectin type III domain 2 (FN2).
[Figure 6] Figure 6 shows the EphA4 cleavage-enhancing activity of the anti-EphA4 monoclonal antibodies (antibodies A and B) in hippocampal neurons.
[Figure 7] Figure 7 shows the EphA4 cleavage-enhancing activity of the anti-EphA4 monoclonal antibodies (antibodies A and B) in hippocampal neurons.
[Figure 8] Figure 8 shows the increasing effect of the anti-EphA4 monoclonal antibodies (antibodies A and B) on the number of spines in hippocampal neurons.

### [Description of Embodiments]

Regions defined or encoded by SEQ ID NOs used in the present specification are as follows:

| SEQ. No. | Sequence region | SEQ. No. | Sequence region |
|---|---|---|---|
| 1 | Full-length human EphA4 (amino acid sequence) | 32 | Heavy chain CDR3 of antibody A (amino acid sequence) |
| 2 | Extracellular domain of human EphA4 (amino acid sequence) | 33 | Light chain CDR1 of antibody A (amino acid sequence) |
| 3 | Human EphA4 extracellular domain-SEAP-His protein (amino acid sequence) | 34 | Light chain CDR2 of antibody A or B (amino acid sequence) |
| 4 | Human EphA4 extracellular domain-Fc-His protein (amino acid sequence) | 35 | Light chain CDR3 of antibody A or B (amino acid sequence) |
| 5 | Human EphA4 extracellular domain-MBP-His protein (amino acid sequence) | 36 | Heavy chain CDR1 of antibody A (nucleic acid sequence) |
| 6 | Signal sequence of human EphA4 (amino acid sequence) | 37 | Heavy chain CDR2 of antibody A (nucleic acid sequence) |
| 7 | Heavy chain variable region of antibody A (amino acid sequence) | 38 | Heavy chain CDR3 of antibody A (nucleic acid sequence) |
| 8 | Light chain variable region of antibody A (amino acid sequence) | 39 | Light chain CDR1 of antibody A (nucleic acid sequence) |
| 9 | Heavy chain variable region of antibody A (nucleic acid sequence) | 40 | Light chain CDR2 of antibody A (nucleic acid sequence) |
| 10 | Light chain variable region of antibody A (nucleic acid sequence) | 41 | Light chain CDR3 of antibody A (nucleic acid sequence) |
| 11 | Heavy chain variable region of antibody B (amino acid sequence) | 42 | Heavy chain CDR1 of antibody B (amino acid sequence) |
| 12 | Light chain variable region of antibody B (amino acid sequence) | 43 | Heavy chain CDR3 of antibody B (amino acid sequence) |
| 13 | Heavy chain variable region of antibody B (nucleic acid sequence) | 44 | Light chain CDR1 of antibody B (amino acid sequence) |
| 14 | Light chain variable region of antibody B (nucleic acid sequence) | 45 | Heavy chain CDR1 of antibody B (nucleic acid sequence) |
| 15 | Heavy chain constant region (human IgG₂) of antibody A (amino acid sequence) | 46 | Heavy chain CDR2 of antibody B (nucleic acid sequence) |
| 16 | Heavy chain constant region (human IgG_{1/2}) of antibody B (amino acid sequence) | 47 | Heavy chain CDR3 of antibody B (nucleic acid sequence) |
| 17 | Light chain constant region (human Igλ) of antibody A or B (amino acid sequence) | 48 | Light chain CDR1 of antibody B (nucleic acid sequence) |
| 18 | Heavy chain constant region of antibody A (nucleic acid sequence) | 49 | Light chain CDR2 of antibody B (nucleic acid sequence) |
| 19 | Light chain constant region of antibody A (nucleic acid sequence) | 50 | Light chain CDR3 of antibody B (nucleic acid sequence) |
| 20 | Full-length heavy chain of antibody A (amino acid sequence) | 51 | Full-length monkey EphA4 (amino acid sequence) |
| 21 | Full-length light chain of antibody A (amino acid sequence) | 52 | Extracellular domain of monkey EphA4 (amino acid sequence) |
| 22 | Full-length heavy chain of antibody A (nucleic acid sequence) | 53 | Full-length rat EphA4 (amino acid sequence) |
| 23 | Full-length light chain of antibody A (nucleic acid sequence) | 54 | Extracellular domain of rat EphA4 (amino acid sequence) |
| 24 | Heavy chain constant region of antibody B (nucleic acid sequence) | 55 | Full-length mouse EphA4 (amino acid sequence) |
| 25 | Light chain constant region of antibody B (nucleic acid sequence) | 56 | Extracellular domain of mouse EphA4 (amino acid sequence) |
| 26 | Full-length heavy chain of antibody B (amino acid sequence) | 57 | Signal sequence of preprotrypsin (amino acid sequence) |
| 27 | Full-length light chain of antibody B (amino acid sequence) | 58 | Ligand-binding domain of human EphA4 (amino acid sequence) |
| 28 | Full-length heavy chain of antibody B (nucleic acid sequence) | 59 | Fibronectin type III domain 1 of human EphA4 (amino acid sequence) |
| 29 | Full-length light chain of antibody B (nucleic acid sequence) | 60 | Fibronectin type III domain 2 of human EphA4 (amino acid sequence) |
| 30 | Heavy chain CDR1 of antibody A (amino acid sequence) | 61 | MBP-His protein (amino acid sequence) |
| 31 | Heavy chain CDR2 of antibody A or B (amino acid sequence) | | |

The present disclosure relates to an anti-EphA4 antibody that binds to EphA4.

The anti-EphA4 antibody according to the present disclosure is an antibody that can recognize and bind to EphA4. As mentioned below, the antibody may be an intact antibody or may be a synthetic antibody (e.g., a recombinant antibody, a chimeric antibody, and a humanized antibody) as long as it has binding affinity to EphA4. In the present specification, it can be understood that EphA4 refers to human-, mouse-, rat-, or monkey-derived EphA4. The human-, mouse-, rat-, or monkey-derived EphA4 can be obtained from a public database in which sequence information is registered, such as GenBank provided by National Center for Biotechnology Information (USA). Alternatively, EphA4 gene sequence information can be obtained by designing primers based on the nucleotide sequence information of EphA4 of a closely related animal species, and then cloning from RNA extracted from the desired animal species. For example, nucleotide sequence information on human, mouse, rat, or monkey EphA4 is registered under GenBank Accession Nos. NM_004438.5, NM_007936.3, NM_001162411.1, and NM_001260870.1, respectively, on the database.

In one aspect, the anti-EphA4 antibody is an antibody that specifically binds to EphA4. The term "specific binding" is a term well known to those skilled in the art, and a method for determining the specific binding of an antibody or an antigen-binding fragment thereof to an antigen or an epitope is also well known. In one embodiment, it is understood that the "specific binding" means that the anti-EphA4 antibody is capable of binding to EphA4 through immunological reaction with larger binding affinity and binding activity, more rapidly and/or for a duration of a longer time as compared with its binding to other target molecules. This does not mean that the antibody that specifically binds to EphA4 does not bind to another target molecule. In another embodiment, the "specific binding" can be indicated by an antibody having Kd against EphA4 of at least approximately 10⁻⁷ M, at least approximately 10⁻⁸ M, or at least approximately 10⁻⁹ M or lower. In a further alternative embodiment, it is understood that the "specific binding" is binding to EphA4 through immunological reaction, but not substantially binding to other family molecules of Eph receptors.

In one aspect, the anti-EphA4 antibody is an antibody that binds to an extracellular domain of EphA4. In one embodiment, the anti-EphA4 antibody is an antibody that binds to a ligand-binding domain (LBD) in the extracellular domain of EphA4.

In one embodiment, the anti-EphA4 antibody can specifically bind to EphA4 and enhance the cleavage of EphA4. In a particular embodiment, the anti-EphA4 antibody can specifically bind to EphA4 and enhance the cleavage of an EphA4 extracellular domain by matrix metalloproteinase (MMP) or ADAM (a disintegrin and metalloproteinase).

In one embodiment, the anti-EphA4 antibody can specifically bind to EphA4 and inhibit the binding between EphA4 and its ligand ephrin.

In another embodiment, the anti-EphA4 antibody can specifically bind to EphA4 and increase the number of spines in hippocampal neurons or stabilize spines in hippocampal neurons.

In one embodiment, the present disclosure encompasses an anti-EphA4 antibody that can specifically bind to at least one of human EphA4, mouse EphA4, rat EphA4, and monkey EphA4 and inhibit the binding thereof to its ligand. In another embodiment, the present disclosure encompasses an anti-EphA4 antibody that can specifically bind to two or more of human EphA4, mouse EphA4, rat EphA4, and monkey EphA4 and inhibit the binding thereof to their ligands. In a further alternative embodiment, the present disclosure encompasses an anti-EphA4 antibody that can specifically bind to all of human EphA4, mouse EphA4, rat EphA4, and monkey EphA4 and inhibit the binding thereof to their ligands.

A method well-known to those skilled in the art can be used as a method for measuring the antigen-binding properties (e.g., binding affinity and cross-species reactivity) of the anti-EphA4 antibody. For example, the binding affinity can be measured by use of, but not limited to, Biacore(R) biosensor, KinExA biosensor, scintillation proximity assay, ELISA, ORIGEN immunoassay (IGEN International, Inc.), flow cytometry, fluorescence quenching, fluorescence transfer, yeast display, and/or immunostaining. The neutralizing activity of the anti-EphA4 antibody against the binding between EphA4 and its ligand can be measured by use of, but not limited to, Biacore(R) biosensor, ELISA, and/or flow cytometry.

The anti-EphA4 antibody according to the present disclosure may be a monoclonal antibody as long as it binds to EphA4.

The anti-EphA4 antibody according to the present disclosure can be of any class such as IgG, IgA or IgM (or subclass thereof) and is not limited to a particular class. Immunoglobulins are classified into different classes depending on the antibody amino acid sequences of their heavy chain (may be referred to as H-chain) constant regions. There are five main immunoglobulin classes: IgA, IgD, IgE, IgG, and IgM, some of which can be further divided into subclasses (isotypes) of, for example, IgG₁, IgG₂, IgG₂, IgG₄, IgA₂, and IgA₂. The heavy chain constant regions corresponding to different classes of immunoglobulins are respectively called α, δ, ε, γ, and µ. The light chain (may be referred to as L-chain) types of antibodies are λ and κ chains.

The anti-EphA4 antibody according to the present disclosure may be an IgG antibody and may be, for example, an IgG₁ antibody or an IgG₂ antibody. Also, the anti-EphA4 antibody according to the present disclosure may be a monomer, a dimer, or a multimer in some instances.

The anti-EphA4 antibody according to the present disclosure may be a combination of IgG antibodies of different subclasses and may be, for example, a human IgG antibody consisting of a combination of IgG₁ and IgG₂ (in the present disclosure, also referred to as human IgG_{1/2}).

The variable region of the antibody according to the present disclosure may mean a variable region of an antibody light chain and/or a variable region of an antibody heavy chain, and the constant region of the antibody may mean a constant region of an antibody light chain and/or a constant region of an antibody heavy chain. The heavy chain and light chain variable regions are each composed of four framework regions (FRs) connected via three CDRs also known as complementarity-determining regions. The CDRs in each chain are held in close proximity by FRs and contribute, together with CDRs in the other chain, to the formation of the antigen-binding site of the antibody. The Kabat numbering system (Kabat et al., Sequences of Proteins of Immunological Interest, 5th ed., 1991, National Institutes of Health, Bethesda MD), which is an approach based on cross-species sequence variability, is used as a technique for determining CDRs.

In one embodiment, the anti-EphA4 antibody according to the present disclosure comprises a heavy chain constant region and/or a light chain constant region derived from a human antibody.

In the present specification, the monoclonal antibody may mean an antibody that is obtained from a population of substantially homogeneous antibodies. Specifically, individual antibodies contained in the population are identical except for natural mutants that might be present to some extent. The monoclonal antibody is directed to a single antigen site and is very specific. In contrast to a typical polyclonal antibody targeting different antigens or different epitopes, each monoclonal antibody targets a single epitope in an antigen. The modifier "monoclonal" denotes the characteristics of the antibody that is obtained from a population of substantially homogeneous antibodies, and should not be restrictively interpreted as requiring the production of the antibody by a particular method.

In the present disclosure, the human antibody means an antibody having the sequences of variable regions and constant regions derived from human immunoglobulin sequences and encompasses an antibody comprising a sequence allowed to contain the desired modification, for example, engineering to increase binding affinity to a target, to reduce immunogenicity, or to increase stability as well as engineering to reduce the inhomogeneity of antibodies produced by non-human cells, as long as it is derived from a human immunoglobulin sequence.

The anti-EphA4 antibody according to the present disclosure also includes an antibody derived from the human antibody by appropriate engineering (e.g., the modification of the antibody or the partial substitution, addition, and/or deletion of the amino acid sequence of the antibody) such that the antibody maintains its functions (or a function is imparted to the antibody or a function of the antibody is improved). More specifically, an antibody having an engineered amino acid sequence of a constant region in order to modify the effector functions of the antibody is also included in the scope of the present disclosure. For example, an antibody derived from a human IgG₂ antibody by the substitution of valine (Val) at position 234 (Eu numbering) with alanine (Ala) and the substitution of glycine (Gly) at position 237 (Eu numbering) with alanine (Ala) in order to reduce antibody-dependent cellular cytotoxicity (ADCC) activity and/or antibody-dependent cellular phagocytosis (ADCP) activity is also included in the scope of the present disclosure. Furthermore, a bispecific antibody having both of an antigen-binding site having the CDR sequences of the anti-EphA4 antibody according to the present disclosure and an antigen-binding site that binds to a different antigen (Kontermann (2012), mAbs 4, 182-97) is also included in the scope of the present disclosure.

The human antibody can be produced by a method known in the art. The human antibody of the present disclosure can be produced as a recombinant antibody, for example, by obtaining an antibody having binding affinity to EphA4 from a known phage display human antibody library (naive library, synthetic library, etc.), determining the sequences of its variable regions, and transferring the sequences in combination with the sequences of the desired human constant regions to non-human animal cells.

Alternatively, the human antibody can be produced by immunizing any of many non-human transgenic animals (containing some or all of the gene loci of a human immunoglobulin heavy chain and light chain in their genomes) with an EphA4 antigen. In one embodiment, a non-human animal containing the human immunoglobulin gene is an animal having human immunoglobulin "minigene loci" (e.g., GenPharm International, Inc.). In some embodiments, the human antibody can be produced using XenoMouse(R) mouse (Abgenix, Inc., Fremont, CA), HuMAb-Mouse(R) mouse (Medarex, Inc.), VelocImmune(R) mouse (Regeneron Pharmaceuticals, Inc.), AlivaMab mouse (Ablexis, LLC), or the like.

The anti-EphA4 antibody according to the present disclosure may be modified, if desired. The modification of the anti-EphA4 antibody may be a modification that changes (a) the three-dimensional structure of an amino acid sequence in a modification region, such as sheet or helix conformation; (b) the electric charge or hydrophobic status of the molecule at a target site; or (c) the effects of a modification on the maintenance of side chain volume, or may be a modification by which these changes are not clearly observed.

The modification of the anti-EphA4 antibody according to the present disclosure may be achieved by, for example, the substitution, deletion, and/or addition of a constituent amino acid residue(s).

In the present specification, the amino acid is used in the broadest sense thereof and includes not only natural amino acids, for example, serine (Ser), asparagine (Asn), valine (Val), leucine (Leu), isoleucine (Ile), alanine (Ala), tyrosine (Tyr), glycine (Gly), lysine (Lys), arginine (Arg), histidine (His), aspartic acid (Asp), glutamic acid (Glu), glutamine (Gln), threonine (Thr), cysteine (Cys), methionine (Met), phenylalanine (Phe), tryptophan (Trp), and proline (Pro) but non-natural amino acids such as amino acid variants and derivatives. Those skilled in the art naturally understand, by taking this wide definition into consideration, that examples of the amino acid in the present specification include: L-amino acids; D-amino acids; chemically modified amino acids such as amino acid variants and amino acid derivatives; amino acids, such as norleucine, β-alanine, and ornithine, which do not serve as materials constituting proteins *in vivo;* and chemically synthesized compounds having the characteristics of amino acids well-known to those skilled in the art. Examples of the non-natural amino acids include α-methylamino acids (α-methylalanine, etc.), D-amino acids (D-aspartic acid, D-glutamic acid, etc.), histidine-like amino acids (2-amino-histidine, β-hydroxy-histidine, homohistidine, α-fluoromethyl-histidine, α-methyl-histidine, etc.), amino acids having extra methylene in their side chains ("homo" amino acids), and amino acids in which a carboxylic acid functional group in the side chain is replaced with a sulfonic acid group (cysteic acid, etc.).

Naturally occurring amino acid residues can be classified into, for example, the following groups based on general side chain characteristics:
(1) hydrophobic residues: Met, Ala, Val, Leu, and Ile;
(2) neutral hydrophilic residues: Asn, Gln, Cys, Ser, and Thr;
(3) acidic residues: Asp and Glu;
(4) basic residues: His, Lys, and Arg;
(5) residues influencing chain orientation: Gly and Pro; and
(6) aromatic residues: Trp, Tyr, and Phe.

The non-conservative substitution of an amino acid sequence constituting the antibody may be performed by replacing an amino acid belonging to one of these groups with an amino acid belonging to any of the other groups. More conservative substitution may be performed by replacing an amino acid belonging to one of these groups with another amino acid belonging to the same group thereas. Likewise, the deletion or the substitution in an amino acid sequence may be appropriately performed.

The modification of amino acid(s) constituting the antibody may be, for example, a posttranslational modification such as glycosylation with a sugar, acetylation, or phosphorylation. The antibody may be glycosylated at a conserved position in its constant region. The glycosylation of the antibody is usually of N-linked or O-linked type. The N-linked glycosylation means the binding of a carbohydrate moiety to the side chain of an asparagine residue. Tripeptide sequences asparagine-X-serine, asparagine-X-threonine, and asparagine-X-cysteine (wherein X is any amino acid other than proline) are recognition sequences for enzymatically adding a carbohydrate moiety to the asparagine side chain. Any of these tripeptide sequences are present in the antibody so that a potential glycosylation site is present. The O-linked glycosylation may be the binding of N-acetylgalactosamine, galactose, or xylose to a hydroxyamino acid (e.g., serine or threonine), or may be the binding thereof to 5-hydroxyproline or 5-hydroxylysine in some instances. Those skilled in the art can appropriately select the glycosylation conditions (in the case of performing the glycosylation by use of a biological approach, for example, host cells and the type and pH of a cell medium) according to the purpose.

The anti-EphA4 antibody according to the present disclosure can be further modified by using other modification methods singly or in combination on the basis of the common technical knowledge well-known to those skilled in the art.

The anti-EphA4 antibody according to the present disclosure can be produced by a method well known to those skilled in the art. For example, a nucleic acid encoding the anti-EphA4 antibody according to the present disclosure may be integrated into an expression vector. The expression vector may be transferred to host cells. The host cells can be cultured to produce an antibody. Accordingly, the present disclosure encompasses a nucleic acid encoding the anti-EphA4 antibody, a vector comprising the nucleic acid, a host cell comprising the vector, and a method for producing an anti-EphA4 antibody, comprising the step of culturing the host cell.

The nucleic acid encoding the anti-EphA4 antibody according to the present disclosure may have DNA encoding a signal sequence and may have DNA encoding a signal sequence at each of the 5' ends of DNA encoding the heavy chain variable region and DNA encoding the light chain variable region. The signal sequence is amino acid residues located at the N-terminal of a protein, which are required for a secretory protein or an integral membrane protein to pass through the lipid bilayer after being synthesized on the ribosome. The signal sequence according to the present disclosure is not particularly limited as long as the sequence has this function. Examples of the signal sequence that may be contained in the anti-EphA4 antibody according to the present disclosure include signal sequences derived from a human, a mouse, a rat, a rabbit, a donkey, a goat, a horse, a chicken, a dog, a cat, a yeast, and the like.

The anti-EphA4 antibody according to the present disclosure may be isolated or purified according to a method well-known to those skilled in the art.

In the present specification, the term "isolated" or "purified" means being artificially isolated or purified from a natural state. When a naturally occurring molecule or composition is altered or removed from its original environment, or both, the molecule or the composition is "isolated" or "purified". Examples of the isolation or purification method include electrophoretic, molecular biological, immunological, and chromatographic approaches and specifically include, but are not limited to, ion-exchange chromatography, hydrophobic chromatography, reverse-phase HPLC chromatography, isoelectric focusing electrophoresis, and alkali extraction.

In one embodiment, the anti-EphA4 antibody comprises the following CDRs:
(a) a heavy chain CDR1 consisting of the amino acid sequence shown in SEQ ID NO: 30;
(b) a heavy chain CDR2 consisting of the amino acid sequence shown in SEQ ID NO: 31;
(c) a heavy chain CDR3 consisting of the amino acid sequence shown in SEQ ID NO: 32;
(d) a light chain CDR1 consisting of the amino acid sequence shown in SEQ ID NO: 33;
(e) a light chain CDR2 consisting of the amino acid sequence shown in SEQ ID NO: 34; and
(f) a light chain CDR3 consisting of the amino acid sequence shown in SEQ ID NO: 35.

In one embodiment, the anti-EphA4 antibody comprises the following CDRs:
(g) a heavy chain CDR1 consisting of the amino acid sequence shown in SEQ ID NO: 42;
(h) a heavy chain CDR2 consisting of the amino acid sequence shown in SEQ ID NO: 31;
(i) a heavy chain CDR3 consisting of the amino acid sequence shown in SEQ ID NO: 43;
(j) a light chain CDR1 consisting of the amino acid sequence shown in SEQ ID NO: 44;
(k) a light chain CDR2 consisting of the amino acid sequence shown in SEQ ID NO: 34; and
(l) a light chain CDR3 consisting of the amino acid sequence shown in SEQ ID NO: 35.

In one embodiment, the anti-EphA4 antibody is a human antibody.

In another embodiment, the anti-EphA4 antibody comprises a heavy chain and a light chain. The heavy chain comprises a variable region consisting of the amino acid sequence shown in SEQ ID NO: 7 or 11, and/or the light chain comprises a variable region consisting of the amino acid sequence shown in SEQ ID NO: 8 or 12. In this embodiment, the variable region of the heavy chain and/or the variable region of the light chain may comprise an amino acid sequence derived from the amino acid sequence shown in SEQ ID NO: 7 or 11 and/or the amino acid sequence shown in SEQ ID NO: 8 or 12 by the substitution, addition, and/or deletion of one or more amino acids. In this context, the term "or more" used as to EphA4 is not limited as long as the resulting sequence maintains binding affinity to EphA4 and enhances the cleavage of EphA4. The term "or more" is 2 to 15 or 2 to 10, for example, 9, 8, 7, 6, 5, 4, 3 or 2, or is within 10%, for example, within 9%, within 8%, within 7%, within 6%, within 5%, within 4%, within 3%, within 2% or within 1%, of the number of amino acids in the amino acid sequence.

In a particular embodiment, the anti-EphA4 antibody comprises a heavy chain and a light chain. The heavy chain comprises a variable region consisting of the amino acid sequence shown in SEQ ID NO: 7, and the light chain comprises a variable region consisting of the amino acid sequence shown in SEQ ID NO: 8.

In a particular embodiment, the anti-EphA4 antibody comprises a heavy chain and a light chain. The heavy chain comprises a variable region consisting of the amino acid sequence shown in SEQ ID NO: 11, and the light chain comprises a variable region consisting of the amino acid sequence shown in SEQ ID NO: 12.

In one embodiment, the heavy chain of the anti-EphA4 antibody comprises a constant region of human IgG₂. In a particular embodiment, the constant region of the human IgG₂ comprises the amino acid sequence of SEQ ID NO: 15.

In another embodiment, the heavy chain of the anti-EphA4 antibody comprises a constant region of human IgG consisting of a combination of human IgG₁ and IgG₂. In a particular embodiment, the constant region of the human IgG consisting of a combination of human IgG₁ and IgG₂ comprises the amino acid sequence of SEQ ID NO: 16.

In one embodiment, the light chain of the anti-EphA4 antibody comprises a constant region of human Igλ. In a particular embodiment, the constant region of the human Igλ comprises the amino acid sequence of SEQ ID NO: 17.

In one embodiment, the heavy chain of the anti-EphA4 antibody comprises the amino acid sequence shown in SEQ ID NO: 20, and the light chain of the anti-EphA4 antibody comprises the amino acid sequence shown in SEQ ID NO: 21.

In another embodiment, the heavy chain of the anti-EphA4 antibody comprises the amino acid sequence shown in SEQ ID NO: 20, the light chain of the anti-EphA4 antibody comprises the amino acid sequence shown in SEQ ID NO: 21, and a C-terminal lysine of the heavy chain may be optionally deleted.

In a particular embodiment, the heavy chain of the anti-EphA4 antibody comprises the amino acid sequence shown in SEQ ID NO: 20, the light chain of the anti-EphA4 antibody comprises the amino acid sequence shown in SEQ ID NO: 21, and a C-terminal lysine of the heavy chain is deleted.

In one embodiment, the heavy chain of the anti-EphA4 antibody comprises the amino acid sequence shown in SEQ ID NO: 26, and the light chain of the anti-EphA4 antibody comprises the amino acid sequence shown in SEQ ID NO: 27.

In another embodiment, the heavy chain of the anti-EphA4 antibody comprises the amino acid sequence shown in SEQ ID NO: 26, the light chain of the anti-EphA4 antibody comprises the amino acid sequence shown in SEQ ID NO: 27, and a C-terminal lysine of the heavy chain may be optionally deleted.

In a particular embodiment, the heavy chain of the anti-EphA4 antibody comprises the amino acid sequence shown in SEQ ID NO: 26, the light chain of the anti-EphA4 antibody comprises the amino acid sequence shown in SEQ ID NO: 27, and a C-terminal lysine of the heavy chain is deleted.

In an alternative embodiment, the anti-EphA4 antibody may have lysine positioned at the C-terminal (carboxy terminal) of the heavy chain deleted for the reason that, for example, the inhomogeneity of antibodies produced by antibody-producing cells is reduced (U.S. Patent Application Publication No. 2010/0297697; and Liu H et al., MAbs. 2014 Sep-Oct; 6 (5): 1145-1154). In the present disclosure, the anti-EphA4 antibody having C-terminal lysine of the heavy chain deleted also includes an anti-EphA4 antibody having C-terminal lysine of the heavy chain deleted by gene engineering, and an anti-EphA4 antibody having C-terminal lysine of the heavy chain deleted by posttranslational cleavage by carboxypeptidase or the like. In the present disclosure, the anti-EphA4 antibody having C-terminal lysine of the heavy chain deleted also includes an anti-EphA4 antibody having C-terminal lysine deleted in both the heavy chains as well as an anti-EphA4 antibody having C-terminal lysine deleted in only one of the heavy chains.

In one aspect, the present disclosure relates to an isolated nucleic acid encoding the anti-EphA4 antibody. The isolated nucleic acid encoding the anti-EphA4 antibody refers to one or more nucleic acid molecules encoding the heavy chain and/or the light chain of the anti-EphA4 antibody. In one embodiment, the nucleic acid according to the present disclosure encodes the heavy chain of the anti-EphA4 antibody. In another embodiment, the nucleic acid according to the present disclosure encodes the light chain of the anti-EphA4 antibody. In a further alternative embodiment, the nucleic acid according to the present disclosure encodes the heavy chain and the light chain of the anti-EphA4 antibody. The nucleic acid according to the present disclosure also includes a first nucleic acid molecule encoding the heavy chain of the anti-EphA4 antibody, and a second nucleic acid molecule encoding the light chain of the anti-EphA4 antibody.

In another aspect, the present disclosure relates to a vector comprising the isolated nucleic acid encoding the anti-EphA4 antibody. The vector according to the present disclosure refers to one or more vectors comprising the isolated nucleic acid encoding the anti-EphA4 antibody. In one embodiment, the vector according to the present disclosure is a vector comprising the nucleic acid encoding the heavy chain of the anti-EphA4 antibody and the nucleic acid encoding the light chain of the anti-EphA4 antibody. In another embodiment, the vector according to the present disclosure is a vector comprising the nucleic acid encoding the heavy chain and the light chain of the anti-EphA4 antibody. In a further alternative embodiment, the vector according to the present disclosure includes a first vector comprising the nucleic acid encoding the heavy chain of the anti-EphA4 antibody, and a second vector comprising the nucleic acid encoding the light chain of the anti-EphA4 antibody. The vector according to the present disclosure can be, but not particularly limited to, a plasmid, a cosmid, a virus, a phage, or the like. For example, a virus vector such as a retrovirus, lentivirus, adenovirus, adeno-associated virus or herpes simplex virus vector is also included in the vector according to the present disclosure.

In a further alternative aspect, the present disclosure also includes a host cell comprising the vector according to the present disclosure, and a method for producing an anti-EphA4 antibody, comprising the step of culturing the host cell. The host cell according to the present disclosure can be, but not particularly limited to, an *E. coli* cell, a monkey COS cell, a Chinese hamster ovary (CHO) cell, a NS0 cell, or the like. In one embodiment, the method for producing an anti-EphA4 antibody comprises the steps of: culturing the host cell; and recovering the anti-EphA4 antibody secreted from the host cell (or a culture medium of the host cell).

In one aspect, the present disclosure relates to a pharmaceutical composition comprising the anti-EphA4 antibody. The pharmaceutical composition according to the present disclosure can be produced according to a known method, for example, a method described in the Japanese Pharmacopeia (JP), the U.S. Pharmacopeia (USP) or the European Pharmacopeia (EP).

The anti-EphA4 antibody according to the present disclosure can be useful in the treatment of Alzheimer's disease. Specifically, in one aspect, the present disclosure encompasses a pharmaceutical composition for the treatment of Alzheimer's disease, comprising the anti-EphA4 antibody. Another aspect encompasses a method for treating Alzheimer's disease, comprising the step of administering a therapeutic effective amount of the anti-EphA4 antibody to a subject having Alzheimer's disease. In an alternative aspect, the present disclosure encompasses use of the anti-EphA4 antibody for producing a therapeutic drug for Alzheimer's disease. In an alternative aspect, the present disclosure encompasses the anti-EphA4 antibody for use in the treatment of Alzheimer's disease.

The anti-EphA4 antibody according to the present disclosure can be used alone or in combination with other drugs or compositions in the treatment method. For example, the anti-EphA4 antibody according to the present disclosure may be administered at the same time or at different times with an additional drug. Such combination therapy includes combined administration (two or more drugs are contained in the same formulation or separate formulations) and separated administration (e.g., simultaneous or sequential). In the case of separately administering two or more drugs, the anti-EphA4 antibody according to the present disclosure can be administered prior to or following the concomitant treatment method.

The subject for administering the pharmaceutical composition according to the present disclosure is not limited, and the pharmaceutical composition can be used for, for example, a human or a non-human mammal (a monkey, a mouse, a rat, a rabbit, cattle, a horse, a goat, etc.).

The method for administering the pharmaceutical composition according to the present disclosure to the subject (administration route, dose, the number of doses per day, the timing of administration, etc.) is not limited and can be appropriately determined by those skilled in the art (e.g., a physician) according to the health condition of the subject, the severity of the disease, the type of a drug to be used in combination therewith, etc.

Those skilled in the art should understand that the present disclosure may be carried out by any one of or appropriate combination of two or more of all aspects described in the present specification unless a technical contradiction arises. Further, those skilled in the art should understood that the present disclosure can be preferably carried out by an appropriate combination of all preferable or advantageous aspects described in the present specification unless a technical contradiction arises.

Literatures cited in the present specification should be interpreted as being incorporated herein by reference in their entirety. Those skilled in the art can understand related contents disclosed in these literatures by reference as a part of the present specification without departing from the spirits and scope of the present disclosure according to the context of the present specification.

Literatures cited in the present specification are provided merely for the purpose of disclosing related techniques before the filing date of the present application. It should not be interpreted that the present inventors admit to having no right preceding such disclosure due to the prior inventions or any other reasons. All statements of these literatures are based on information which has been available by the present applicant, and there is no admission that the contents of these statements are accurate.

The terms in the present specification are used for illustrating particular embodiments and are not intended to limit the invention.

The term "comprise" used in the present specification means that described items (members, steps, factors, numbers, etc.) are present and the presence of the other items (members, steps, factors, numbers, etc.) is not excluded therefrom, unless the context evidently requires different interpretation. The term "consist of" encompasses aspects described by the terms "consist of" and/or "consist essentially of".

The term "neutralizing activity" used in the present specification means the activity of inhibiting the binding between EphA4 and its ligand and/or the activity of inhibiting signal transduction or the molecular expression response or functional change of cells induced by the binding between EphA4 and its ligand in the living body of a human.

All terms (including technical terms and scientific terms) used herein have the same meanings as those understood in a broad sense by those skilled in the art to which the present disclosure belongs, unless otherwise defined. The terms used herein should be interpreted as having meanings consistent with meanings in the present specification and related technical fields and should not be interpreted in an idealized or excessively formal sense, unless otherwise defined.

Terms such as "first" or "second" are used for expressing various factors. However, these factors are understood to be not limited by these terms themselves. These terms are used merely for differentiating one factor from the other factors. For example, the first factor may be described as the second factor, and vice versa, without departing from the scope of the present disclosure.

In the present specification, it should be understood that numerical values used for indicating component contents, numerical ranges, etc., are modified with the term "approximately" unless otherwise specified. For example, "4°C" is interpreted as meaning "approximately 4°C" unless otherwise specified. Those skilled in the art can naturally understand the extent thereof rationally according to the technical common sense and the context of the present specification.

It should be understood that each aspect indicated in a singular form used in the present specification and claims may be in a plural form, and vice versa, unless the context evidently requires different interpretation and unless a technical contradiction arises.

Hereinafter, the present disclosure will be described in more detail with reference to Examples. However, the present disclosure can be embodied by various aspects and is not intended to be limited by Examples described herein. Those skilled in the art can implement the present disclosure by various changes or modifications, additions, deletions, substitutions, etc., without departing from the spirit or scope of the present disclosure.

### [Examples]

### Example 1: Preparation of human anti-EphA4 monoclonal antibody

In order to prepare a monoclonal antibody that binds to human EphA4 (GenBank Accession No. NP_004429.1, SEQ ID NO: 1), a protein of a human EphA4 extracellular domain (positions 20 to 547) (SEQ ID NO: 2) fused with secreted alkaline phosphatase (SEAP) and histidine tag (hereinafter, referred to as "human EphA4 extracellular domain-SEAP-His protein", SEQ ID NO: 3), a protein of a human EphA4 extracellular domain fused with a human IgG₁ Fc region (Fc) and histidine tag (hereinafter, referred to as "human EphA4 extracellular domain-Fc-His protein", SEQ ID NO: 4), and a protein of a human EphA4 extracellular domain fused with maltose-binding protein (MBP) and histidine tag (hereinafter, referred to as "human EphA4 extracellular domain-MBP-His protein", SEQ ID NO: 5) were prepared by the following steps.

First, a pcDNA3.1-human EphA4 extracellular domain-SEAP-His expression vector, a pcDNA3.1-human EphA4 extracellular domain-Fc-His expression vector and a pcDNA3.4-human EphA4 extracellular domain-MBP-His expression vector were constructed. First, a DNA sequence encoding the signal sequence (SEQ ID NO: 6) and the extracellular domain of human EphA4 was amplified by RT-PCR using human brain-derived total RNA and cloned into the SalI/NotI site of a pENTR1A vector (Invitrogen/Life Technologies Corp.) having a DNA sequence encoding SEAP and histidine tag or a pENTR1A vector (Invitrogen/Life Technologies Corp.) having a DNA sequence encoding Fc and histidine tag. Next, the DNA sequence encoding the signal sequence and the extracellular domain of human EphA4, SEAP, and histidine tag, or the DNA sequence encoding the signal sequence and the extracellular domain of human EphA4, Fc, and histidine tag was transferred to a pcDNA3.1_rfcB vector through LR reaction using Gateway System (Invitrogen/Life Technologies Corp.) to construct a pcDNA3.1-human EphA4 extracellular domain-SEAP-His expression vector and a pcDNA3.1-human EphA4 extracellular domain-Fc-His expression vector. As for the pcDNA3.4-human EphA4 extracellular domain-MBP-His expression vector, a DNA sequence encoding the signal sequence and the extracellular domain of human EphA4 was amplified by PCR and cloned into a pcDNA3.4 vector (Invitrogen/Life Technologies Corp.) having a DNA sequence encoding MBP and histidine tag to construct a human EphA4 extracellular domain-MBP-His protein expression vector. Expi293F cells (Thermo Fisher Scientific Inc.) were transfected with each of expression vectors described above using Expi293 expression system (Thermo Fisher Scientific Inc.). Four days later, each culture solution was recovered and clarified by the removal of the cells. The resulting culture solution was purified using TALON resin (Takara Bio Inc.) and buffer-replaced with PBS (FUJIFILM Wako Pure Chemical Corp.) by dialysis.

Screening was carried out using the human EphA4 protein and a fully human antibody synthetic phage library to obtain a human antibody fragment (scFv) that specifically binds to human EphA4. The human EphA4 extracellular domain-SEAP-His protein was captured onto Dynabeads magnetic beads (Thermo Fisher Scientific Inc.) or a nickel plate (Pierce Biotechnology Inc.), and the fully human antibody synthetic phage library was added thereto. One hour or two hours later, unbound phages were removed by a series of washing cycles using PBS-Tween (0.1% v/v) or PBS. Bound phage particles were eluted and then amplified via the infection of *E. coli* TG1 host cells. The infected TG1 cells were harvested, seeded over a plate, and incubated at 30°C. This panning treatment was further performed twice using the amplified phages.

After three rounds of panning, a single colony from the TG1 cells infected with the enriched phages was seeded to a medium in a 96-well plate. The expression of FLAG-tagged scFv was induced by the addition of IPTG, followed by shake culture overnight at 30°C. The TG1 cells were spun down, and wells having reactivity with human EphA4 were picked up using the *E. coli* culture supernatant containing scFv.

The reactivity with human EphA4 was evaluated by ELISA according to the following steps using the human EphA4 extracellular domain-Fc-His protein or the human EphA4 extracellular domain-MBP-His protein. Each well of a 96-well plate (Nunc) was coated with an anti-FLAG antibody (Sigma-Aldrich Co. LLC). After incubation overnight at 4°C, each well was blocked with 2% skim milk (Becton Dickinson and Company) at room temperature for 2 hours. After washing with 0.02% Tween 20/PBS (Nacalai Tesque, Inc.) three times, the human EphA4 extracellular domain-Fc-His protein or the human EphA4 extracellular domain-MBP-His protein (final concentration: 20 nM) and the *E. coli* culture supernatant containing scFv were added to each well and incubated at room temperature for 2 hours. After washing three times, a horseradish peroxidase-labeled anti-His antibody (Medical & Biological Laboratories Co., Ltd.) was added thereto and incubated at room temperature for 1 hour. After washing five times, TMBZ (3,3',5,5'-tetramethylbenzidine, Kirkegaard & Perry Laboratories, Inc.) solution was added to each well and incubated at room temperature for 15 to 20 minutes. An equal amount of a reaction stopping solution (1 N H₂SO₄, FUJIFILM Wako Pure Chemical Corp.) was added to each well. The absorbance at 450 nm was read using a microplate reader (Thermo Fisher Scientific Inc.). As a result of screening, human antibody fragments specific for human EphA4 were selected, and the gene sequence of each fragment was determined by sequencing.

DNA sequences encoding the variable regions of each obtained human antibody fragment (scFv) were subcloned into a vector for the expression of antibody heavy chain and light chain constant regions to convert the clone from scFv into an IgG form. Expi293F cells (Thermo Fisher Scientific Inc.) were transfected with the expression vector (pcDNA3.4) containing the gene sequence encoding the human anti-EphA4 monoclonal antibody using Expi293 expression system (Thermo Fisher Scientific Inc.). A supernatant was recovered, and the human anti-EphA4 monoclonal antibody was obtained using MabSelectSuRe (Cytiva). The human anti-EphA4 monoclonal antibody candidates thus obtained were narrowed down from the viewpoint of cleavage-enhancing activity against EphA4, specificity for EphA4, the phosphorylated state of a downstream molecule of EphA4 signals, immunogenicity, etc. The immunogenicity was evaluated using EpiScreen(R) (Abzena pic). In this series of evaluations, antibodies obtained by the shuffling the heavy chains and the light chains of the human anti-EphA4 monoclonal antibodies and antibodies having mutations to CDRs were also prepared, and a total of 300 or more human anti-EphA4 monoclonal antibodies were evaluated.

The EphA4 cleavage-enhancing activity of each obtained human anti-EphA4 monoclonal antibody was evaluated using rat hippocampal neurons. The rat hippocampal neurons were prepared according to the following steps. A fetus was taken out of a rat (Charles River Laboratories Japan, Inc.) in 18 days of pregnancy, and the head was incised to isolate the brain. A hippocampal region was excised therefrom under a stereomicroscope, then placed in a digestion solution (137 mM NaCl (FUJIFILM Wako Pure Chemical Corp.), 5 mM KCl (FUJIFILM Wako Pure Chemical Corp.), 7 mM Na₂HPO₄ (FUJIFILM Wako Pure Chemical Corp.), 25 mM Hepes (Dojindo Laboratories), 0.5 mg/mL DNase (Sigma-Aldrich Co. LLC), 0.25% trypsin (Life Technologies Corp.)), and shaken at 37°C for 10 minutes. The solution was removed, and 20% fetal bovine serum/Hanks buffer solution (Sigma-Aldrich Co. LLC) was added to the residue. After removal of the liquid and washing with a Hanks buffer solution twice, the hippocampal tissues were pipetted in a Hanks buffer solution to prepare a cell suspension. The cells were seeded in a poly-L lysine-coated 96-well dish (Falcon) containing a culture solution (Neurobasal medium (Life Technologies Corp.), 1 × B-27 supplement (Life Technologies Corp.), 0.5 mM L-glutamine (Life Technologies Corp.)).

The EphA4 cleavage-enhancing activity evaluation using the hippocampal neurons was conducted according to the following steps. The rat hippocampal neurons seeded in a 96-well dish (Falcon) were treated with the anti-EphA4 monoclonal antibody (20 nM) and a γ-secretase inhibitor Compound E (50 nM, Enzo Life Sciences, Inc.), and 24 hours later, washed with PBS (FUJIFILM Wako Pure Chemical Corp.). The cells were harvested by the addition of an SDS sample buffer (Laemmli sample buffer (Bio-Rad Laboratories, Inc.), 5% 2-mercaptoethanol (Bio-Rad Laboratories, Inc.)) and boiled for 5 minutes. Analysis by SDS-PAGE and Western blotting or analysis using fully automated Western system Jess (ProteinSimple Inc.) were conducted using an anti-EphA4 monoclonal antibody (Abnova Corp.). Band intensity was quantified, and the value of EphA4 C-terminal fragment/full-length EphA4 was then calculated.

An antibody A and an antibody B were obtained as human anti-EphA4 monoclonal antibodies having the activity of enhancing the cleavage of EphA4 by the EphA4 cleavage-enhancing activity evaluation described above. The antibody A and the antibody B were antibodies that did not cause phosphorylation of a downstream molecule of EphA4 signals and had low immunogenicity (both T cell proliferation and IL-2 production were 10% or less).

Genes encoding the full-length heavy chains and light chains of the antibody A and the antibody B were totally synthesized at GenScript. The amino acid sequence of the heavy chain variable region of the antibody A is the amino acid sequence shown in SEQ ID NO: 7, and the amino acid sequence of the light chain variable region thereof is the amino acid sequence shown in SEQ ID NO: 8. As a gene sequence encoding the amino acid sequence of the antibody A, the nucleic acid sequence shown in SEQ ID NO: 9 was used for the heavy chain variable region, and the nucleic acid sequence shown in SEQ ID NO: 10 was used for the light chain variable region. The amino acid sequence of the heavy chain variable region of the antibody B is the amino acid sequence shown in SEQ ID NO: 11, and the amino acid sequence of the light chain variable region thereof is the amino acid sequence shown in SEQ ID NO: 12. As a gene sequence encoding the amino acid sequence of the antibody B, the nucleic acid sequence shown in SEQ ID NO: 13 was used for the heavy chain variable region, and the nucleic acid sequence shown in SEQ ID NO: 14 was used for the light chain variable region. A human IgG₂ constant region (SEQ ID NO: 15) was used as the heavy chain constant region of the antibody A, and a human IgG_{1/2} constant region (SEQ ID NO: 16) having human IgG₁ CH1 and hinges and human IgG₂ CH2 and CH3 was used as the heavy chain constant region of the antibody B. Human IgX (SEQ ID NO: 17) was used as the light chain constant regions of the antibody A and the antibody B. As a gene sequence encoding the amino acid sequence of the antibody A, the nucleic acid sequence shown in SEQ ID NO: 18 was used for the heavy chain constant region, and the nucleic acid sequence shown in SEQ ID NO: 19 was used for the light chain constant region. The amino acid sequence of the full-length heavy chain (excluding the signal sequence) of the antibody A is the amino acid sequence shown in SEQ ID NO: 20, and the amino acid sequence of the full-length light chain (excluding the signal sequence) thereof is the amino acid sequence shown in SEQ ID NO: 21. A nucleic acid sequence encoding the full-length heavy chain of the antibody A is the nucleic acid sequence shown in SEQ ID NO: 22, and a nucleic acid sequence encoding the full-length light chain thereof is the nucleic acid sequence shown in SEQ ID NO: 23. As a gene sequence encoding the amino acid sequence of the antibody B, the nucleic acid sequence shown in SEQ ID NO: 24 was used for the heavy chain constant region, and the nucleic acid sequence shown in SEQ ID NO: 25 was used for the light chain constant region. The amino acid sequence of the full-length heavy chain (excluding the signal sequence) of the antibody B is the amino acid sequence shown in SEQ ID NO: 26, and the amino acid sequence of the full-length light chain (excluding the signal sequence) thereof is the amino acid sequence shown in SEQ ID NO: 27. A nucleic acid sequence encoding the full-length heavy chain of the antibody B is the nucleic acid sequence shown in SEQ ID NO: 28, and a nucleic acid sequence encoding the full-length light chain thereof is the nucleic acid sequence shown in SEQ ID NO: 29. Expi293F cells (Thermo Fisher Scientific Inc.) or CHOK1SV cells (Lonza) were transfected with an expression vector (pcDNA3.4 or pEE6.4 and pEE12.4) containing the gene sequence encoding the amino acid sequence of the antibody A or the antibody B using Expi293 expression system (Thermo Fisher Scientific Inc.) or GS system (Lonza). A supernatant was recovered, and the antibody A and the antibody B were purified as human anti-EphA4 monoclonal antibodies using MabSelectSuRe (Cytiva).

The CDRs of the antibody A and the antibody B were determined according to the Kabat definition for the identification of CDRs (Kabat numbering system). The amino acid sequences and the nucleic acid sequences of the CDRs of the antibody A are shown in Tables 1 and 2, respectively. The amino acid sequences and the nucleic acid sequences of the CDRs of the antibody B are shown in Tables 3 and 4, respectively.

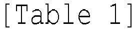

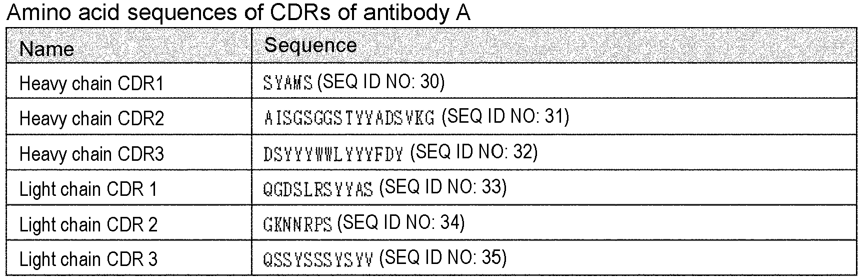

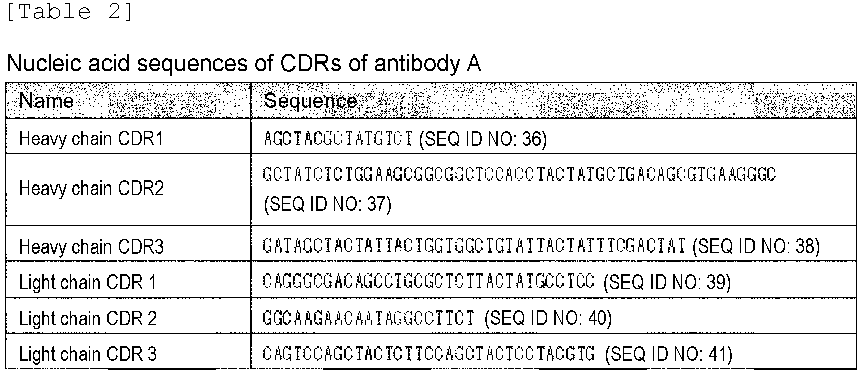

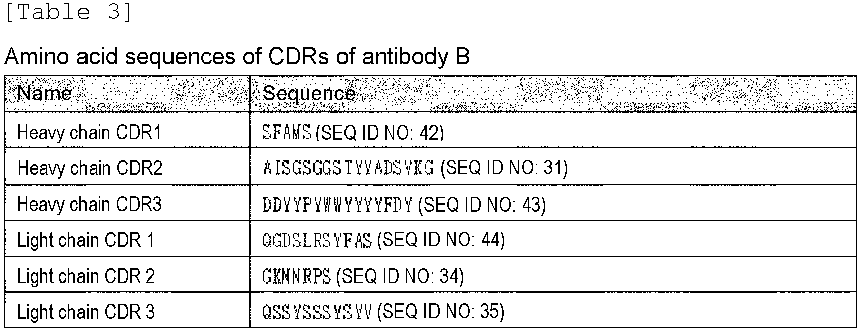

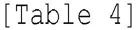

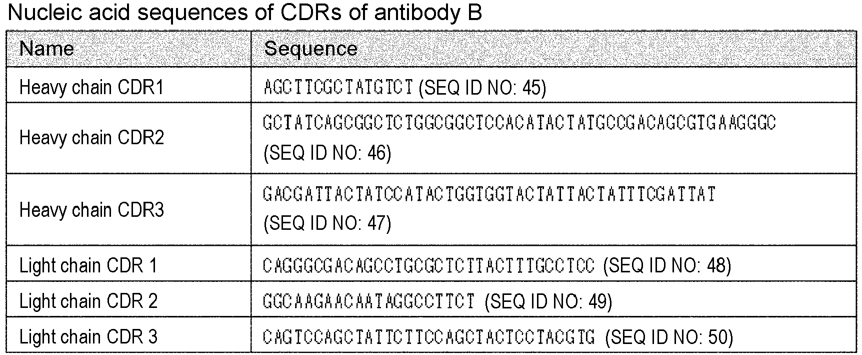

### Example 2: Binding affinity of human anti-EphA4 monoclonal antibody to human EphA4

The binding affinity of the antibody A and the antibody B to human EphA4 was determined by surface plasmon resonance (SPR) using Biacore T200 (Cytiva). First, an anti-His antibody (Cytiva, 28-9950-56) was immobilized on sensor chip CM5. The immobilization was performed by the amine coupling method using N-hydroxysuccinimide (NHS) and N-ethyl-N'-(3-dimethylaminopropyl)carbodiimide hydrochloride (EDC). Ethanolamine was used in blocking (the sensor chip and the reagents for immobilizations were all manufactured by Cytiva). The antibody was diluted with a buffer solution for immobilization (10 mM sodium acetate, pH 4.5) into 3 µg/mL and immobilized on the sensor chip according to the protocol attached to Biacore T200. Human EphA4 extracellular domain-SEAP-His10 was diluted with a running buffer solution HBS-EP+ (Cytiva, BR-1001-69), injected onto a flow cell for 120 seconds, and captured (amount of the antibody captured: approximately 6 RU). Subsequently, the antibody A or the antibody B serially diluted in the range of 100, 50, 25, 12.5, 6.3, 3.2, 1.6, and 0 nM using HBS-EP+ was added to the sensor chip for 120 seconds. Binding reaction curves were sequentially observed at the time of the addition (association phase, 120 sec) and after the completion of the addition (dissociation phase, 600 sec). After the completion of each observation, the sensor chip was regenerated by the addition of 3 M MgCl₂ (60 sec). The obtained binding reaction curves were subjected to fitting analysis using 1:1 binding models and software BIA evaluation attached to the system to calculate the binding affinity (KD = kd / ka) to human EphA4. The experiment mentioned above was carried out three times, and an average value of each parameter was calculated.

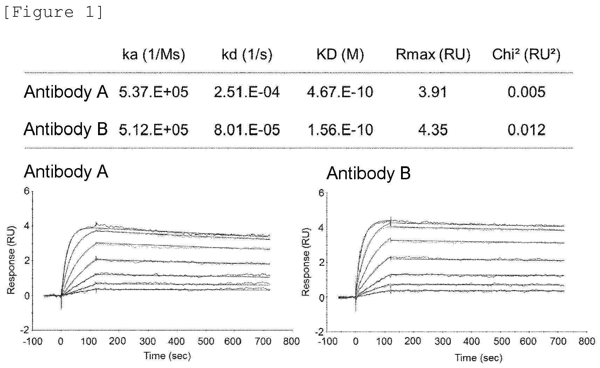

The binding affinity (KD value) of the antibody A and the antibody B to human EphA4 was 4.67 × 10⁻¹⁰ M and 1.56 × 10⁻¹⁰ M, respectively (Figure 1). The antibody A and the antibody B had the same level of binding affinity to human EphA4. Figure 1 shows a typical binding reaction curve as an example.

### Example 3: Human EphA4-human ligand binding inhibitory activity of human anti-EphA4 monoclonal antibody

The antibody A and the antibody B were evaluated for their inhibitory activity against the binding between human EphA4 and its human ligand according to the following steps. Each well of a 96-well plate (Nunc) was coated with an anti-alkaline phosphatase antibody (Thermo Fisher Scientific Inc.). After incubation overnight at 4°C, each well was blocked with 1% BlockAce (KAC Co., Ltd.) overnight at 4°C. After washing with 0.02% Tween 20/PBS three times, the human EphA4 extracellular domain-SEAP-His protein (final concentration: 10 nM) was seeded in each well and incubated at room temperature for 1 hour. After washing three times, ligands and serially diluted antibody A or antibody B (0, 0.003, 0.01, 0.03, 0.1, 0.3, 1, 3, 10, 30, 100, 300, 1000, and 3000 nM) were added to each well. The ligands used were biotinylated human Ephrin A5-Fc chimera (R&D Systems, Inc., final concentration: 0.7 nM) and biotinylated human Ephrin B3-Fc chimera (R&D Systems, Inc., final concentration: 2.3 nM). After incubation at room temperature for 1 hour and subsequent washing three times, a horseradish peroxidase-labeled streptavidin (GE Healthcare Japan Corp.) was added thereto and incubated at room temperature for 1 hour. After washing three times, a TMBZ (3,3',5,5'-tetramethylbenzidine, Kirkegaard & Perry Laboratories, Inc.) solution was added to each well and incubated at room temperature for 15 to 20 minutes. An equal amount of a reaction stopping solution (2 N H₂SO₄, FUJIFILM Wako Pure Chemical Corp.) was added to each well. The absorbance at 450 nm was read using a microplate reader (Thermo Fisher Scientific Inc.).

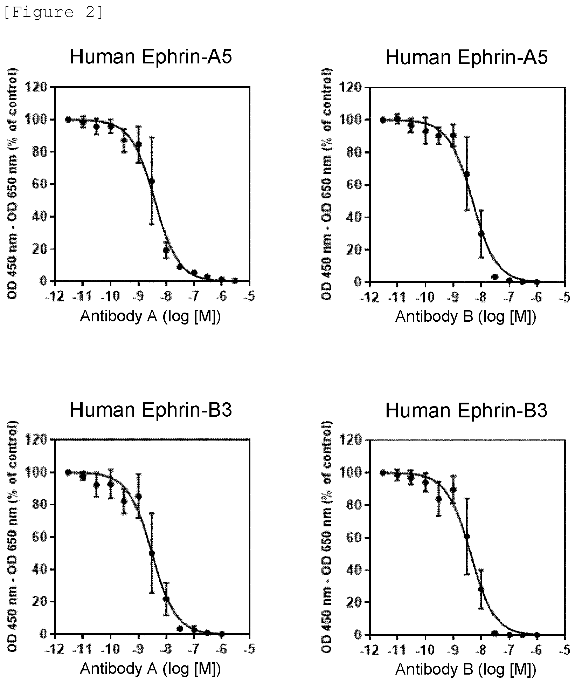

The antibody A and the antibody B suppressed the binding between human EphA4 and its human ligand in a concentration-dependent manner. The IC₅₀ values of the antibody A for binding to human Ephrin A5 and Ephrin B3 were 3.9 nM and 3.0 nM, respectively, and the IC₅₀ values of the antibody B for binding to human Ephrin A5 and Ephrin B3 were 4.8 nM and 4.1 nM, respectively. Accordingly, the antibody A and the antibody B were found to inhibit the binding between human EphA4 and the human ligand (Figure 2).

### Example 4: Selectivity of human anti-EphA4 monoclonal antibody for human Eph receptor

The antibody A and the antibody B were evaluated for their binding activity against each human Eph receptor according to the following steps. Each well of a 96-well plate (Nunc) was coated with a rabbit anti-6-His antibody (Bethyl Laboratories, Inc.). After incubation at room temperature for 1 hour or overnight at 4°C, each well was blocked with 1% BlockAce (KAC Co., Ltd.) at room temperature for 1 hour. After washing with 0.02% Tween 20/PBS three times, each human Eph receptor extracellular domain-His protein (Creative BioMart Inc., final concentration: 1 nM) was seeded in each well and incubated at room temperature for 1 hour. After washing three times, the antibody A or the antibody B (10 µg/mL) was added to each well and incubated at room temperature for 1 hour. After washing three times, a horseradish peroxidase-labeled rabbit anti-human IgG Fcy fragment antibody (Jackson ImmunoResearch Laboratories, Inc.) was added thereto and incubated at room temperature for 1 hour. After washing three times, a TMBZ (3,3',5,5'-tetramethylbenzidine, Kirkegaard & Perry Laboratories, Inc.) solution was added to each well. An equal amount of a reaction stopping solution (2 N H₂SO₄, FUJIFILM Wako Pure Chemical Corp.) was added to each well. The absorbance at 450 nm was read using a microplate reader (Thermo Fisher Scientific Inc.).

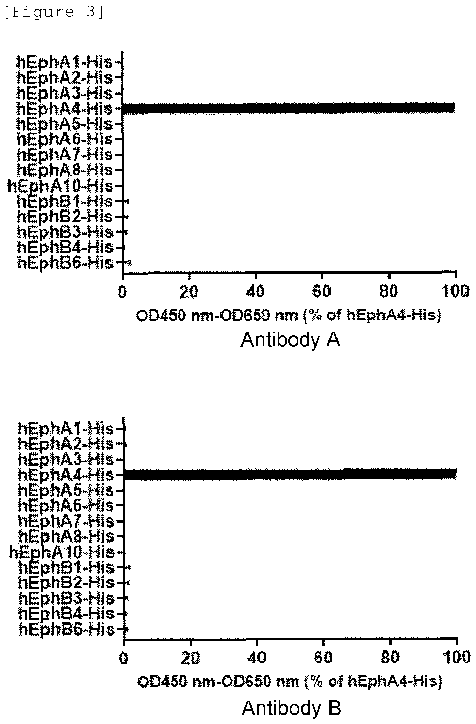

The antibody A and the antibody B were found to specifically bind to human EphA4 among the members of the human Eph receptor family (Figure 3).

### Example 5: Reactivity of human anti-EphA4 monoclonal antibody with mouse, rat, monkey or human EphA4

Mouse, rat, monkey and human EphA4 extracellular domain-SEAP-His proteins were prepared according to the following steps. Genes encoding SEAP-His and mouse, rat, monkey and human EphA4 extracellular domains were synthesized at GenScript Japan Inc.. First, the synthesized gene fragment encoding SEAP-His was cloned into a pcDNA3.4 vector (Invitrogen/Life Technologies Corp.). Each of the gene fragments of the mouse, rat, monkey and human EphA4 extracellular domains was cloned into the constructed pcDNA3.4-SEAP-His expression vector to construct mouse, rat, monkey and human EphA4 extracellular domain-SEAP-His expression vectors. The amino acid sequence of the human EphA4 used in the vector construction is shown in SEQ ID NO: 1, and its extracellular domain is shown in SEQ ID NO: 2. The amino acid sequence of the monkey EphA4 is shown in SEQ ID NO: 51, and its extracellular domain is shown in SEQ ID NO: 52. The amino acid sequence of the rat EphA4 is shown in SEQ ID NO: 53, and its extracellular domain is shown in SEQ ID NO: 54. The amino acid sequence of the mouse EphA4 is shown in SEQ ID NO: 55, and its extracellular domain is shown in SEQ ID NO: 56. Various EphA4 extracellular domain-SEAP-His proteins were prepared using the human EphA4 extracellular domain-SEAP-His protein expression vector, the monkey EphA4 extracellular domain-SEAP-His protein expression vector, the rat EphA4 extracellular domain-SEAP-His protein expression vector, and the mouse EphA4 extracellular domain-SEAP-His protein expression vector. Expi293F cells (Thermo Fisher Scientific Inc.) were transfected with each of the expression vectors using Expi293 expression system (Thermo Fisher Scientific Inc.). Four days later, each culture solution was recovered and clarified by the removal of the cells. The resulting culture solution was purified using TALON resin (Takara Bio Inc.) and buffer-replaced with PBS (FUJIFILM Wako Pure Chemical Corp.) by dialysis.

The antibody A and the antibody B were evaluated for their binding activity against each EphA4 according to the following steps. Each well of a 96-well plate (Nunc) was coated with a rabbit anti-6-His antibody (Bethyl Laboratories, Inc.). After incubation at room temperature for 1 hour, each well was blocked with 1% BlockAce (KAC Co., Ltd.) overnight at 4°C. After washing with 0.02% Tween 20/PBS three times, each of the mouse, rat, monkey and human EphA4 extracellular domain-SEAP-His proteins (final concentration: 1 nM) was seeded in each well and incubated at room temperature for 1 hour. After washing three times, the antibody A or the antibody B (0, 0.00013, 0.00064, 0.0032, 0.016, 0.08, 0.4, 2, and 10 µg/mL) was added to each well and incubated at room temperature for 1 hour. After washing three times, a horseradish peroxidase-labeled rabbit anti-human IgG Fcy fragment antibody (Jackson ImmunoResearch Laboratories, Inc.) was added thereto and incubated at room temperature for 1 hour. After washing three times, a TMBZ (3,3',5,5'-tetramethylbenzidine, Kirkegaard & Perry Laboratories, Inc.) solution was added to each well. An equal amount of a reaction stopping solution (2 N H₂SO₄, FUJIFILM Wako Pure Chemical Corp.) was added to each well. The absorbance at 450 nm was read using a microplate reader (Thermo Fisher Scientific Inc.).

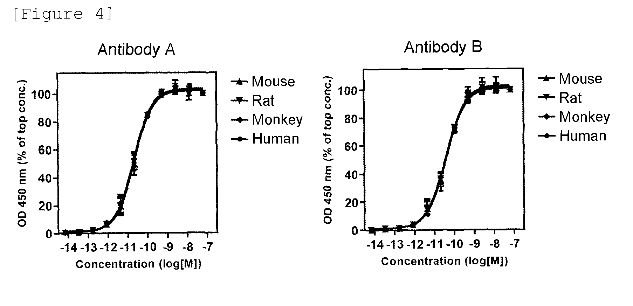

The antibody A and the antibody B had the same level of binding activity against all of mouse EphA4, rat EphA4, monkey EphA4 and human EphA4 (Figure 4).

### Example 6: Reactivity of human anti-EphA4 monoclonal antibody with human EphA4 extracellular domain, ligand-binding domain, fibronectin type III domain 1, and fibronectin type III domain 2

A protein of a human EphA4 extracellular domain (ECD), ligand-binding domain (LBD), fibronectin type III domain 1 (FN1) or fibronectin type III domain 2 (FN2) fused with maltose-binding protein (MBP) and histidine tag (hereinafter, referred to as "human EphA4 extracellular domain-MBP-His protein", "human EphA4 ligand-binding domain-MBP-His protein", "human EphA4 fibronectin type III domain 1-MBP-His protein", and "human EphA4 fibronectin type III domain 2-MBP-His protein") was prepared according to the following steps. First, a pcDNA3.4-human EphA4 extracellular domain-, ligand-binding domain-, fibronectin type III domain 1-, or fibronectin type III domain 2-MBP-His expression vector was constructed. First, a DNA sequence encoding the signal sequence (SEQ ID NO: 6) of human EphA4 or the signal sequence (SEQ ID NO: 57) of preprotrypsin and each domain of human EphA4 was amplified by PCR and cloned into a pcDNA3.4 vector (Invitrogen/Life Technologies Corp.) having a DNA sequence encoding MBP and histidine tag with an AAA or G4S linker to construct human EphA4 extracellular domain-MBP-His protein, human EphA4 ligand-binding domain-MBP-His protein, human EphA4 fibronectin type III domain 1-MBP-His protein, and human EphA4 fibronectin type III domain 2-MBP-His protein expression vectors. The amino acid sequence of the human EphA4 used in the vector construction is shown in SEQ ID NO: 1; its extracellular domain is shown in SEQ ID NO: 2; the ligand-binding domain is shown in SEQ ID NO: 58; the fibronectin type III domain 1 is shown in SEQ ID NO: 59; and the fibronectin type III domain 2 is shown in SEQ ID NO: 60. MBP and histidine tag (MBP-His protein) are shown in SEQ ID NO: 61. Expi293F cells (Thermo Fisher Scientific Inc.) were transfected with each of the expression vectors using Expi293 expression system (Thermo Fisher Scientific Inc.). Four days later, each culture solution was recovered and clarified by the removal of the cells. The human EphA4 extracellular domain-MBP-His protein or the human EphA4 ligand-binding domain-MBP-His protein was purified using TALON resin (Takara Bio Inc.) and buffer-replaced with PBS (FUJIFILM Wako Pure Chemical Corp.) by dialysis. The human EphA4 fibronectin type III domain 1-MBP-His protein and the human EphA4 fibronectin type III domain 2-MBP-His protein were purified using Amylose resin (New England Biolabs Inc.), and monomer fractions were separated and purified using AKTA Explore 10s/Superdex 200 10/300 GL (Cytiva).

The antibody A, the antibody B, and human IgG (Sigma-Aldrich Co. LLC) were evaluated for their binding activity against each EphA4 according to the following steps. Each well of a 96-well plate (Nunc) was coated with a rabbit anti-6-His antibody (Bethyl Laboratories, Inc.). After incubation at room temperature for 1 hour, each well was blocked with 1% BlockAce (KAC Co., Ltd.) overnight at 4°C. After washing with 0.02% Tween 20/PBS three times, each of the human EphA4 extracellular domain-, human EphA4 ligand-binding domain-, human EphA4 fibronectin type III domain 1-, and human EphA4 fibronectin type III domain 2-MBP-His proteins or the MBP-His protein (final concentration: 1 nM) was seeded in each well and incubated at room temperature for 1 hour. After washing three times, the antibody A, the antibody B, or human IgG (10 nM) was added to each well and incubated at room temperature for 1 hour. After washing three times, a horseradish peroxidase-labeled rabbit anti-human IgG Fcy fragment antibody (Jackson ImmunoResearch Laboratories, Inc.) was added thereto and incubated at room temperature for 1 hour. After washing three times, a TMBZ (3,3',5,5'-tetramethylbenzidine, Kirkegaard & Perry Laboratories, Inc.) solution was added to each well. An equal amount of a reaction stopping solution (2 N H₂SO₄, FUJIFILM Wako Pure Chemical Corp.) was added to each well. The absorbance at 450 nm and 650 nm was read using a microplate reader (Thermo Fisher Scientific Inc.).

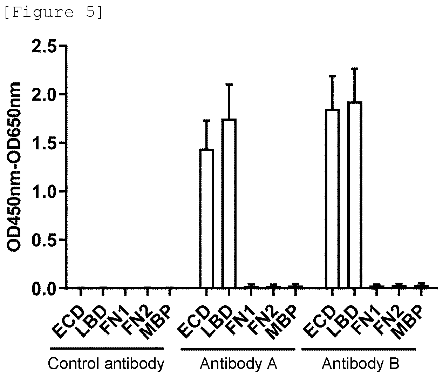

The antibody A and the antibody B had binding activity against the human EphA4 extracellular domain (ECD) and ligand-binding domain (LBD) (Figure 5). Neither of them reacted with the fibronectin type III domain 1 (FN1) and the fibronectin type III domain 2 (FN2). Accordingly, the antibody A and the antibody B were found to specifically bind to the human EphA4 extracellular domain and ligand-binding domain.

### Example 7: EphA4 cleavage-enhancing activity of human anti-EphA4 monoclonal antibody in hippocampal neuron

Rat hippocampal neurons were prepared according to the following steps. A fetus was taken out of a rat (Charles River Laboratories Japan, Inc.) in 18 days of pregnancy, and the head was incised to isolate the brain. A hippocampal region was excised therefrom under a stereomicroscope, then placed in a digestion solution (137 mM NaCl (FUJIFILM Wako Pure Chemical Corp.), 5 mM KCl (FUJIFILM Wako Pure Chemical Corp.), 7 mM Na₂HPO₄ (FUJIFILM Wako Pure Chemical Corp.), 25 mM Hepes (Dojindo Laboratories), 0.5 mg/mL DNase (Sigma-Aldrich Co. LLC), 0.25% trypsin (Thermo Fisher Scientific Inc.)), and shaken at 37°C for 10 minutes. The solution was removed, and 20% fetal bovine serum/Hanks buffer solution (Sigma-Aldrich Co. LLC) was added to the residue. After removal of the liquid and washing with a Hanks buffer solution twice, the hippocampal tissues were pipetted in a Hanks buffer solution to prepare a cell suspension. The cells were further suspended in a culture solution (Neurobasal medium (Thermo Fisher Scientific Inc.), 1 × B-27 supplement (Thermo Fisher Scientific Inc.), 0.5 mM L-glutamine (Thermo Fisher Scientific Inc.)) and then seeded in a poly-L lysine-coated 96-well dish (Falcon).

The antibody A and the antibody B obtained in Example 1 were evaluated for their EphA4 cleavage-enhancing activity using the hippocampal neurons according to the following steps. A solvent (PBS (FUJIFILM Wako Pure Chemical Corp.)), human IgG, the antibody A, or the antibody B (2.0, 6.7, and 20 nM) was allowed to act for 24 hours, together with a γ-secretase inhibitor Compound E (50 nM, Enzo Life Sciences, Inc.), on the rat hippocampal neurons seeded in a 96-well dish (Falcon). After washing with PBS (FUJIFILM Wako Pure Chemical Corp.), the cells were harvested by the addition of an SDS sample buffer (Laemmli sample buffer (Bio-Rad Laboratories, Inc.), 2.5% 2-mercaptoethanol (Bio-Rad Laboratories, Inc.)) and boiled for 5 minutes. This sample was analyzed using fully automated Western system Jess. For the analysis, an anti-EphA4 monoclonal antibody (Abnova Corp.) was used. The signals of an EphA4 C-terminal fragment and full-length EphA4 were quantified, and the value of EphA4 C-terminal fragment/(full-length EphA4 + EphA4 C-terminal fragment) was calculated.

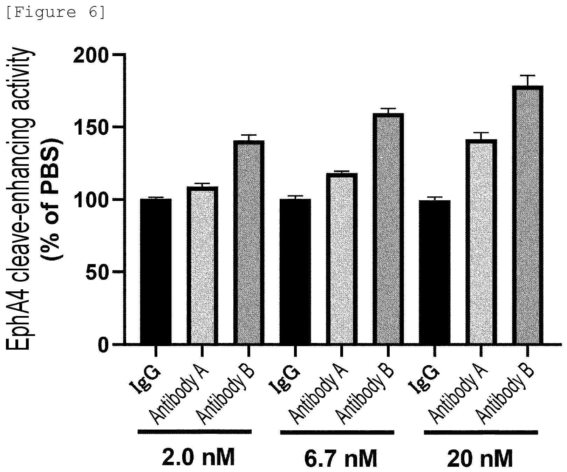

The antibody A and the antibody B enhanced EphA4 cleavage reaction in a concentration-dependent manner in the hippocampal neurons (Figure 6).

### Example 8: Human EphA4 cleavage-enhancing activity of human anti-EphA4 monoclonal antibody

First, a DNA sequence encoding human EphA4 and a pCAHA vector having a DNA sequence encoding HA tag were synthesized at GenScript Japan Inc. Then, a DNA sequence encoding human EphA4 was inserted to the SalI/NotI of the pCAHA vector to construct a pCA-human EphA4-HA expression vector.

Rat hippocampal neurons were prepared according to the following steps. A fetus was taken out of a rat (Charles River Laboratories Japan, Inc.) in 18 days of pregnancy, and the head was incised to isolate the brain. A hippocampal region was excised therefrom under a stereomicroscope, then placed in a digestion solution (137 mM NaCl (FUJIFILM Wako Pure Chemical Corp.), 5 mM KCl (FUJIFILM Wako Pure Chemical Corp.), 7 mM Na₂HPO₄ (FUJIFILM Wako Pure Chemical Corp.), 25 mM Hepes (Dojindo Laboratories), 0.5 mg/mL DNase (Sigma-Aldrich Co. LLC), 0.25% trypsin (Thermo Fisher Scientific Inc.)), and shaken at 37°C for 10 minutes. The solution was removed, and 20% fetal bovine serum/Hanks buffer solution (Sigma-Aldrich Co. LLC) was added to the residue. After removal of the liquid and washing with a Hanks buffer solution twice, the hippocampal tissues were pipetted in a Hanks buffer solution to prepare a cell suspension.

The antibody A and the antibody B obtained in Example 1 were evaluated for their cleavage-enhancing activity against human EphA4 according to the following steps. The pCA-human EphA4-HA expression vector was transferred to the rat hippocampal neurons using Nucleofector (Lonza Group AG). The cells were suspended in a culture solution (Neurobasal medium (Thermo Fisher Scientific Inc.), 1 × B-27 supplement (Thermo Fisher Scientific Inc.), 0.5 mM L-glutamine (Thermo Fisher Scientific Inc.)) and then seeded in a poly-L lysine-coated 96-well dish (Falcon). A solvent (PBS (FUJIFILM Wako Pure Chemical Corp.)), human IgG, the antibody A, or the antibody B (6.7, 20, and 67 nM) was allowed to act overnight, together with a γ-secretase inhibitor Compound E (50 nM, Enzo Life Sciences, Inc.), on the seeded rat hippocampal neurons. After washing with PBS, the cells were harvested by the addition of an SDS sample buffer (Laemmli sample buffer (Bio-Rad Laboratories, Inc.), 5% 2-mercaptoethanol (Bio-Rad Laboratories, Inc.)) and boiled for 5 minutes. This sample was subjected to SDS-PAGE and subjected to Western blotting using a rat anti-HA monoclonal antibody (Roche). Band intensity was quantified, and the value of human EphA4 C-terminal fragment/(full-length human EphA4 + human EphA4 C-terminal fragment) was calculated.

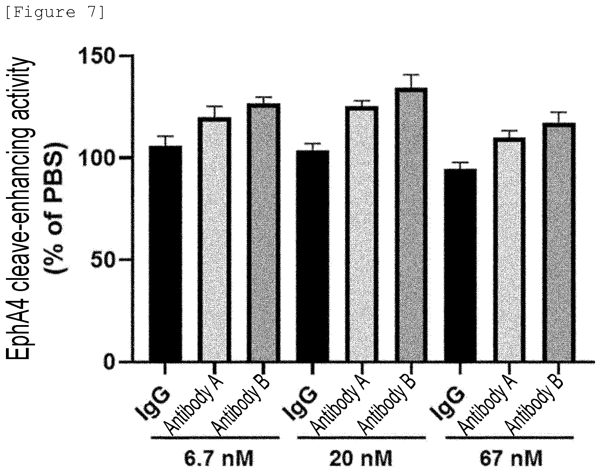

The antibody A and the antibody B enhanced human EphA4 cleavage reaction in the hippocampal neurons (Figure 7).

### Example 9: Increasing effect of human anti-EphA4 monoclonal antibody on spine density in hippocampal neuron

Rat hippocampal neurons were prepared as described in Example 7. EGFP gene was transferred to the rat hippocampal neurons using Nucleofector (Lonza Group AG). The cells were mixed with untransfected neurons, and seeded in poly-L lysine-coated 24-well plates (Falcon) after placement of cover glasses (Matsunami Glass Ind., Ltd.) therein.

Spines were counted with the hippocampal neurons according to the following steps. The rat hippocampal neurons on cultured day 15 seeded in poly-L lysine-coated 24-well plates (Falcon) after placement of cover glasses (Matsunami Glass Ind. Ltd.) therein were treated with a control antibody (human IgG₂; Sigma-Aldrich Co. LLC), the antibody A, or the antibody B (6.7 and 20 nM) for 24 hours. Then, the cover glasses were transferred to 2% PFA (FUJIFILM Wako Pure Chemical Corp.)/4% sucrose (FUJIFILM Wako Pure Chemical Corp.)/PBS and left standing for 20 minutes so that the cells were fixed therein. The cover glasses were taken out of the fixative solution, transferred to PBS, and washed three times. Then, 0.25% Triton X-100 (FUJIFILM Wako Pure Chemical Corp.)/PBS was added thereto, and cell permeation treatment was performed for 15 minutes. The cover glasses were transferred to 2% BSA (Sigma-Aldrich Co. LLC)/0.25% Triton X-100/OPTI-MEM (GIBCO), blocked for 1 hour, and then reacted with an anti-GFP antibody (Nacalai Tesque, Inc.) and an anti-Math2 antibody (Abeam plc) at room temperature for 1 hour to 1.5 hours. The primary antibody solution was removed, and the cover glasses were washed with PBS three times and then reacted with a secondary antibody at room temperature for 1 hour in the dark. The secondary antibody solution was removed, and the cover glasses were washed with PBS three times and then enclosed with Prolong Gold antifade reagent (Molecular Probes, Inc.), followed by observation and the capture of analysis images under LSM800 (Carl Zeiss AG). The experiment mentioned above was carried out three times. Math2-positive pyramidal cell-like neurons were extracted from two cover glasses per experiment, and spines on their respective dendrites were counted by image analysis software Imaris(R) (Bitplane). The number of spines per 10 µm for each neuron was calculated.

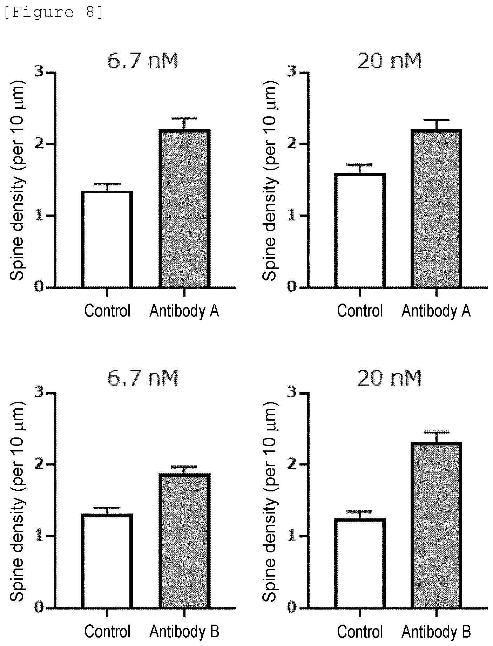

The antibody A and the antibody B increased the spine density of the hippocampal neurons (Figure 8). This result indicates that the antibody A and the antibody B have the ability to stabilize spines in hippocampal neurons.

## Claims

1. An anti-EphA4 antibody comprising
a heavy chain comprising
(a) a heavy chain CDR1 consisting of the amino acid sequence shown in SEQ ID NO: 30;
(b) a heavy chain CDR2 consisting of the amino acid sequence shown in SEQ ID NO: 31; and
(c) a heavy chain CDR3 consisting of the amino acid sequence shown in SEQ ID NO: 32; and
a light chain comprising
(d) a light chain CDR1 consisting of the amino acid sequence shown in SEQ ID NO: 33;
(e) a light chain CDR2 consisting of the amino acid sequence shown in SEQ ID NO: 34; and
(f) a light chain CDR3 consisting of the amino acid sequence shown in SEQ ID NO: 35, or
a heavy chain comprising
(g) a heavy chain CDR1 consisting of the amino acid sequence shown in SEQ ID NO: 42;
(h) a heavy chain CDR2 consisting of the amino acid sequence shown in SEQ ID NO: 31; and
(i) a heavy chain CDR3 consisting of the amino acid sequence shown in SEQ ID NO: 43; and
a light chain comprising
(j) a light chain CDR1 consisting of the amino acid sequence shown in SEQ ID NO: 44;
(k) a light chain CDR2 consisting of the amino acid sequence shown in SEQ ID NO: 34; and
(l) a light chain CDR3 consisting of the amino acid sequence shown in SEQ ID NO: 35.

2. The anti-EphA4 antibody according to claim 1, wherein
the anti-EphA4 antibody is a human antibody.

3. The anti-EphA4 antibody according to claim 1 or 2, wherein
the anti-EphA4 antibody specifically binds to EphA4 and enhances the cleavage of EphA4.

4. The anti-EphA4 antibody according to any one of claims 1 to 3, wherein
the anti-EphA4 antibody specifically binds to EphA4 and inhibits the binding between EphA4 and ephrin.

5. The anti-EphA4 antibody according to any one of claims 1 to 4, wherein
the heavy chain comprises a variable region consisting of the amino acid sequence shown in SEQ ID NO: 7, and
the light chain comprises a variable region consisting of the amino acid sequence shown in SEQ ID NO: 8.

6. The anti-EphA4 antibody according to any one of claims 1 to 5, wherein
a constant region of the heavy chain and a constant region of the light chain each comprise an amino acid sequence derived from a human antibody.

7. The anti-EphA4 antibody according to claim 6, wherein
the constant region of the heavy chain is a constant region of human IgG.

8. The anti-EphA4 antibody according to claim 7, wherein
the constant region of the human IgG is a constant region of human IgG₂.

9. The anti-EphA4 antibody according to claim 8, wherein
the constant region of the human IgG₂ comprises the amino acid sequence shown in SEQ ID NO: 15.

10. The anti-EphA4 antibody according to any one of claims 1 to 4, wherein
the heavy chain comprises a variable region consisting of the amino acid sequence shown in SEQ ID NO: 11, and
the light chain comprises a variable region consisting of the amino acid sequence shown in SEQ ID NO: 12.

11. The anti-EphA4 antibody according to any one of claims 1 to 4 and claim 10, wherein
a constant region of the heavy chain and a constant region of the light chain each comprise an amino acid sequence derived from a human antibody.

12. The anti-EphA4 antibody according to claim 11, wherein
the constant region of the heavy chain is a constant region of human IgG.

13. The anti-EphA4 antibody according to claim 12, wherein
the constant region of the human IgG is a constant region of human IgG consisting of a combination of human IgG₁ and human IgG₂.

14. The anti-EphA4 antibody according to claim 13, wherein
the constant region of the human IgG consisting of a combination of human IgG₁ and human IgG₂ comprises the amino acid sequence shown in SEQ ID NO: 16.

15. The anti-EphA4 antibody according to any one of claims 6 to 9 and claims 11 to 14, wherein
the constant region of the light chain is a constant region of human Igλ.

16. The anti-EphA4 antibody according to claim 15, wherein
the constant region of the human Igλ comprises the amino acid sequence shown in SEQ ID NO: 17.

17. An anti-EphA4 antibody comprising
a heavy chain and a light chain, wherein
the heavy chain comprises the amino acid sequence shown in SEQ ID NO: 20,
the light chain comprises the amino acid sequence shown in SEQ ID NO: 21, and
a C-terminal lysine of the heavy chain may be optionally deleted.

18. The anti-EphA4 antibody according to claim 17, wherein
the C-terminal lysine of the heavy chain is deleted.

19. An anti-EphA4 antibody comprising
a heavy chain and a light chain, wherein
the heavy chain comprises the amino acid sequence shown in SEQ ID NO: 26,
the light chain comprises the amino acid sequence shown in SEQ ID NO: 27, and
a C-terminal lysine of the heavy chain may be optionally deleted.

20. The anti-EphA4 antibody according to claim 19, wherein
the C-terminal lysine of the heavy chain is deleted.

21. An isolated nucleic acid encoding the anti-EphA4 antibody according to any one of claims 1 to 20.

22. A vector comprising the nucleic acid according to claim 21.

23. A host cell comprising the vector according to claim 22.

24. A method for producing an anti-EphA4 antibody, comprising the step of culturing the host cell according to claim 23.
